# EUROPEAN PATENT APPLICATION

(11) **EP 4 119 182 A1**
(43) Date of publication of application: **18.01.2023**
(21) Application number: 21768829.0
(22) Date of filing: 29.01.2021
(51) Int. Cl.: A61M 25/09

(54) **GUIDE WIRE**

(30) Priority: 11.03.2020 JP 2020041613
(71) Applicant: Asahi Intecc Co., Ltd., Seto-shi, Aichi 489-0071 (JP)
(72) Inventor: KOSUGI, Tomoki, Seto-shi, Aichi 489-0071 (JP); TAKAHASHI, Daiki, Seto-shi, Aichi 489-0071 (JP)
(74) Representative: Prinz & Partner mbB
(86) International application number: PCT/JP2021/003202
(87) International publication number: WO 2021/181936

(57) **Abstract**

A guide wire includes a core wire, a first resin layer that coats a part of the core wire, a coil body wound around another part of the core wire and an outer side of the first resin layer, and a second resin layer that coats the coil body, in which the part of the coil body is embedded in the first resin layer and the second resin layer.

## Description

### TECHNICAL FIELD

The disclosed embodiments relate to a guide wire.

### BACKGROUND ART

There is known a guide wire used when a medical device such as a catheter is inserted into a blood vessel. It is desired that such a guide wire has flexibility and resilience to bending, improved slidability with respect to an additional device such as a catheter and an endoscope, and improved safety by making the guide wire resistant to breakage. For example, Patent Literature 1 describes a characteristic in which a guide wire includes an annular member provided to fill a stepped space between a proximal end portion of a resin coating layer and a wire main body, at a proximal end side of the resin coating layer covering an outer periphery of a distal end portion of the wire main body. For example, Patent Literature 2 describes a characteristic in which a guide wire includes a first polymer layer arranged between a core member and a spiral coil, and a second polymer layer arranged around an outer surface 161 of the first polymer layer.

### CITATION LIST

### Patent Literature

Patent Literature 1: JP 5526218 B
Patent Literature 2: JP 2003-513764 W

### SUMMARY OF INVENTION

### TECHNICAL PROBLEM

However, in the guide wire described in Patent Literature 1, an area of a contact surface between the resin coating layer and the annular member is small, and thus, when shear stress along a longitudinal direction of the guide wire is applied, the resin coating layer and the annular member may be possibly separated on such a contact surface. The guide wire described in Patent Literature 2 has a problem in that an entire spiral coil from a distal end to a proximal end is covered with the first polymer layer and the second polymer layer, and thus, flexibility to bending is low. Such a problem is not limited to the vascular system, and is common to a guide wire to be inserted into various organs of a human body, such as the lymphatic system, the biliary system, the urinary system, the respiratory system, the digestive system, a secretory gland, and reproductive organs.

The disclosed embodiments have been contrived to solve at least some of the above-mentioned problems, and an object thereof is to provide a guide wire having flexibility, resistance to breakage, and improved safety.

### SOLUTION TO PROBLEM

The disclosed embodiments have been contrived to solve at least some of the above-described problems, and can be implemented as the following aspects.
(1) According to one aspect of the disclosed embodiments, a guide wire is provided. Such a guide wire is a guide wire including a core wire, a first resin layer that covers a part of the core wire, a coil body wound around another part of the core wire and an outer side of the first resin layer, and a second resin layer that covers the coil body, in which the part of the coil body is embedded in the first resin layer and the second resin layer.

According to such a configuration, the guide wire has a configuration in which the part of the coil body is embedded in the first resin layer and the second resin layer. That is, the guide wire includes, on a contact surface in which each of the first resin layer and the second resin layer contacts the coil body, a portion connected via the coil body (hereinafter, also referred to as "indirectly connected portion") and a portion in which the first resin layer and the second resin layer are directly connected on the contact surface of the first and second resin layers (hereinafter, also referred to as "directly connected portion"). Therefore, as compared to a configuration including only the directly connected portion, it is possible to increase an area of the connected portion (in other words, a contact surface between members contributing to the connection of the first resin layer and the second resin layer) and it is possible to improve a connection strength between the first resin layer and the second resin layer. When the indirectly connected portion in which the first resin layer and the second resin layer are connected via the coil body is provided, even if shear stress along a longitudinal direction of the guide wire is applied, the first resin layer and the second resin layer are not easily separated. Only a part of the coil body is embedded into the first resin layer and the second resin layer, and thus, as compared to a configuration in which the entire coil body from the distal end to the proximal end is embedded in the resin layer, it is possible to provide flexibility to bending. As a result, according to the guide wire of the present embodiment, it is possible to provide a guide wire having flexibility, resistance to breakage, and improved safety.

(2) In the guide wire according to the above aspect, the second resin layer may have a lower hardness than the first resin layer.

According to such a configuration, the second resin layer has a lower hardness than the first resin layer, and thus, it is possible to flexibly configure the second resin layer and the coil body coated with the second resin layer, and it is possible to further improve the flexibility of the guide wire. The hardness of the first resin layer is equal to or higher than the hardness of the second resin layer, and thus, it is possible to cause the first resin layer to function as a protective member that protects a part of the coil body and a part of the core wire.

(3) In the guide wire according to the above aspect, an end portion at a proximal end side of the first resin layer may be located close to a proximal end side of the core wire relative to an end portion at a proximal end side of the second resin layer.

According to such a configuration, the end portion at the proximal end side of the first resin layer is located close to the proximal end side of the core wire relative to the end portion at the proximal end side of the second resin layer. Therefore, it is possible to cause the first resin layer to function as a protective member that protects the end portion at the proximal end side of the coil body and a part of the core wire corresponding to the end portion at the proximal end side of the coil body.

(4) In the guide wire according to the above aspect, the end portion at the proximal end side of the first resin layer may decrease in thickness toward the proximal end side of the core wire.

According to such a configuration, the end portion at the proximal end side of the first resin layer decreases in thickness toward the proximal end side of the core wire, and thus, it is possible to suppress catching on an additional device such as a catheter and an endoscope, and it is possible to improve the slidability with respect to the additional device.

(5) In the guide wire according to the above aspect, the first resin layer may include, at the proximal end side, an inclusion portion having all of the proximal end portion of the coil body being embedded into the first resin layer, and the second resin layer may be arranged only at the distal end side from the distal end of the inclusion portion.

According to such a configuration, the first resin layer includes, at the proximal end side, the inclusion portion having all of the proximal end portion of the coil body being embedded into the first resin layer. Therefore, when the hardness of the first resin layer is configured to be equal to or higher than the hardness of the second resin layer, it is possible to improve a protection capability of protecting the end portion at the proximal end side of the coil body and a part of the core wire corresponding to the proximal end portion of the coil body. Processing for gradually decreasing the thickness of the end portion at the proximal end side of the first resin layer toward the proximal end side, is easier.

(6) In the guide wire according to the above aspect, the core wire may include a protrusion portion having an enlarged diameter at a position contacting the end portion at the proximal end side of the first resin layer.

According to such a configuration, the core wire includes the protrusion portion having an enlarged diameter at a position contacting the end portion at the proximal end side of the first resin layer. Therefore, it is possible to protect the end portion at the proximal end side of the first resin layer by the protrusion portion of the core wire, and it is possible to suppress peeling of the proximal end portion of the first resin layer. It is further possible to regulate a movement of a wire (a wire configuring the coil body) when the shear stress along the longitudinal direction is applied to the guide wire, by the protrusion portion of the core wire. As a result, it is possible to provide a guide wire having enhanced resistance to breakage and improved safety.

(7) In the guide wire according to the above aspect, the protrusion portion may include a reduced diameter portion having an outer diameter decreasing from the proximal end side toward the distal end side, and the end portion at the proximal end side of the first resin layer may be provided in contact with the reduced diameter portion.

According to such a configuration, the end portion at the proximal end side of the first resin layer is provided in contact with the reduced diameter portion of the protrusion portion. Therefore, it is possible to surely protect the end portion at the proximal end side of the first resin layer, and it is possible to reduce a possibility that kinking occurs in the core wire, by suppressing concentration of stress when a shear stress along the longitudinal direction is applied to the guide wire. It is possible to suppress generation of a step as a result of exposure of the core wire at the distal end side from the protrusion portion, and thus, it is possible to suppress catching on the additional device and improve the slidability with respect to the additional device.

(8) The guide wire of the above embodiment may further include a regulation portion arranged between the coil body and the core wire at the distal end side from the first resin layer, the regulation portion having another part of the coil body being embedded therein.

According to such a configuration, the guide wire further includes the regulation portion arranged between the coil body and the core wire at the distal end side from the first resin layer, the regulation portion having another part of the coil body being embedded therein. Thus, when the shear stress along the longitudinal direction is applied to the guide wire, if a movement of the wire (wire configuring the coil body) is regulated by the regulation portion, it is possible to provide a guide wire having enhanced resistance to breakage and improved safety.

It is noted that the disclosed embodiments can be realized in various aspects, and for example, can be realized in an aspect such as a guide wire, a coil body coated with a plurality of resin layers, a method of manufacturing a guide wire, and a method of manufacturing a coil body.

### BRIEF DESCRIPTION OF DRAWINGS

FIG. 1 is an explanatory diagram illustrating an example of a configuration of a guide wire according to a first embodiment.
FIG. 2 is an enlarged view of a part (FIG. 1) of the guide wire.
FIG. 3 is an explanatory diagram illustrating an example of a configuration of a guide wire according to a second embodiment.
FIG. 4 is an explanatory diagram illustrating a part of a proximal end side of the guide wire according to the second embodiment.
FIG. 5 is an explanatory diagram illustrating an example of a configuration of a guide wire according to a third embodiment.
FIG. 6 is an explanatory diagram illustrating an example of a configuration of a guide wire according to a fourth embodiment.
FIG. 7 is an enlarged view illustrating an area in the vicinity of a protrusion portion of the guide wire according to the fourth embodiment.
FIG. 8 is an enlarged view illustrating an area in the vicinity of a protrusion portion of a guide wire according to a comparative example.
FIG. 9 is an enlarged view illustrating an area in the vicinity of a protrusion portion of a guide wire according to a comparative example.
FIG. 10 is an enlarged view illustrating an area in the vicinity of a protrusion portion of a guide wire according to a comparative example.
FIG. 11 is an enlarged view illustrating an area in the vicinity of a protrusion portion of a guide wire according to a comparative example.
FIG. 12 is an explanatory diagram illustrating an example of a configuration of a guide wire according to a fifth embodiment.
FIG. 13 is an explanatory diagram illustrating an example of a configuration of a guide wire according to a sixth embodiment.
FIG. 14 is an explanatory diagram illustrating an example of a configuration of a guide wire according to a seventh embodiment.
FIG. 15 is an explanatory diagram illustrating an example of a configuration of a guide wire according to an eighth embodiment.

### EMBODIMENTS OF THE INVENTION

### <First Embodiment>

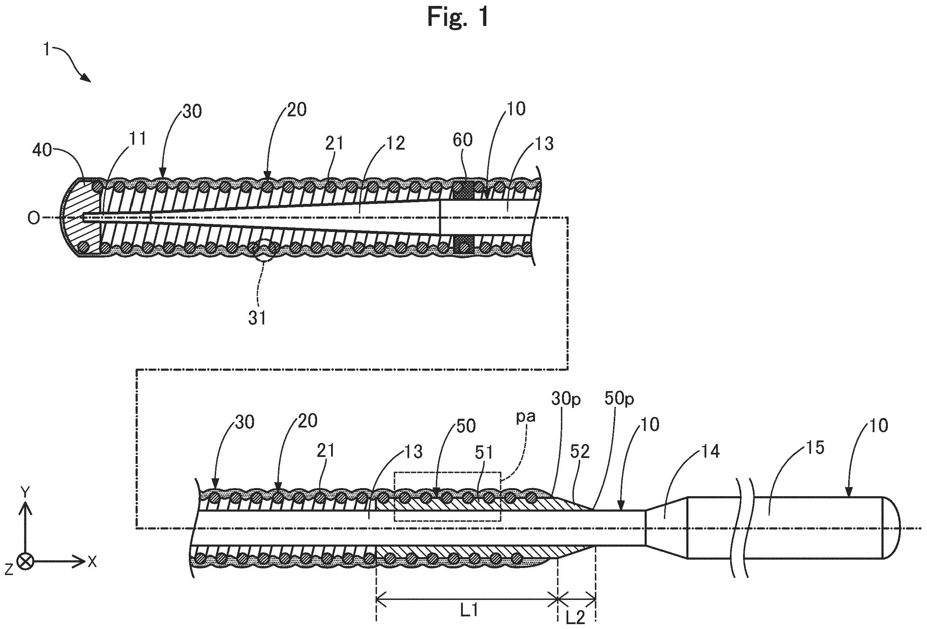

FIG. 1 is an explanatory diagram illustrating an example of a configuration of a guide wire 1 according to a first embodiment. The guide wire 1 is a medical device inserted into a biological lumen in various organs of a human body, such as a vascular system, a lymphatic system, a biliary system, a urinary system, a respiratory system, a digestive system, a secretory gland, and a reproductive organ. The guide wire 1 may be directly inserted into the biological lumen mentioned above, or may be inserted into the biological lumen via an endoscope. The guide wire 1 includes a core wire 10, a coil body 20, a second resin layer 30, a distal end joint portion 40, a first resin layer 50, and an intermediate fixing portion 60.

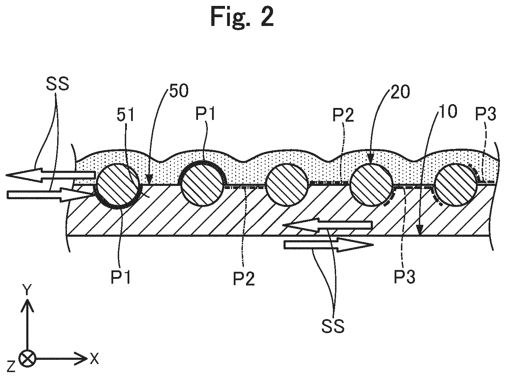

In FIG. 1, an axis passing through a center of the guide wire 1 is represented by an axial line O (dash-dot-dash line). In an example of FIG. 1, the axial line O coincides with axes passing through the guide wire 1 and each center of each constituent member. However, it is not required that the axial line O coincides with central axes of the guide wire 1 and each of the constituent members. FIG. 1 illustrates X-, Y-, and Z-axes orthogonal to one another. The X-axis corresponds to a longitudinal direction of the guide wire 1 (direction of the axial line O), the Y-axis corresponds to a height direction of the guide wire 1, and the Z-axis corresponds to a width direction of the guide wire 1. The left side (- X-axial direction) in FIG. 1 is referred to as "distal end side" of the guide wire 1 and each constituent member, and the right side in FIG. 1 (+ X-axial direction) is referred to as "proximal end side" of the guide wire 1 and each constituent member. In the guide wire 1 and each constituent member, an end portion located at the distal end side is referred to as "distal end", and the distal end and an area in the vicinity thereof are referred to as "distal end portion". Further, an end portion located at the proximal end side is referred to as a "proximal end", and the proximal end and an area in the vicinity thereof are referred to as a "proximal end portion". The distal end side is inserted into a living body, and the proximal end side is operated by an operator such as a doctor. The same applies to FIG. 2 and subsequent drawings.

The core wire 10 is a tapered long member having a large diameter at the proximal end side and a small diameter at the distal end side. The core wire 10 includes a small diameter portion 11, a first reduced diameter portion 12, a first increased diameter portion 13, a second reduced diameter portion 14, and a second increased diameter portion 15, in this order from the distal end side to the proximal end side. An outer diameter and a length at each portion of the core wire 10 may be freely determined.

The small diameter portion 11 is formed at the distal end portion of the core wire 10. The small diameter portion 11 is a portion where an outer diameter of the core wire 10 is smallest, and has a substantially columnar shape having a substantially constant outer diameter. The distal end of the small diameter portion 11 is formed with a distal end joint portion 40. The first reduced diameter portion 12 is formed between the small diameter portion 11 and the first increased diameter portion 13. The first reduced diameter portion 12 has a tapered shape in which the outer diameter decreases from the proximal end side toward the distal end side. The first increased diameter portion 13 is formed between the first reduced diameter portion 12 and the second reduced diameter portion 14. The first increased diameter portion 13 has a substantially columnar shape having a substantially constant outer diameter larger than the outer diameter of the small diameter portion 11. A part of the first increased diameter portion 13 at the proximal end side is coated with the first resin layer 50. The outer peripheral surfaces of the small diameter portion 11, the first reduced diameter portion 12, and the first increased diameter portion 13 are covered by the coil body 20.

The second reduced diameter portion 14 is formed between the first increased diameter portion 13 and the second increased diameter portion 15. The second reduced diameter portion 14 has a tapered shape in which the outer diameter decreases from the proximal end side toward the distal end side. The second increased diameter portion 15 is formed at the proximal end portion of the core wire 10. The second increased diameter portion 15 is a portion where an outer diameter of the core wire 10 is largest, and has a substantially columnar shape having a substantially constant outer diameter. The second reduced diameter portion 14 and the second increased diameter portion 15 are not covered by the coil body 20, and are used when an operator grasps the guide wire 1. An outer diameter and a length of each portion (the small diameter portion 11 to the second increased diameter portion 15) of the core wire 10 may be freely determined.

The coil body 20 is formed by spirally winding a wire 21 around the core wire 10 and the first resin layer 50. The coil body 20 has a substantially cylindrical shape having a substantially constant outer diameter from the proximal end side to the distal end side. The coil body 20 is arranged, at the distal end side, to cover the small diameter portion 11, the first reduced diameter portion 12, and the first increased diameter portion 13 of the core wire 10 with a space therebetween. The coil body 20 is arranged, at the proximal end side, to cover an outer side of the first resin layer 50 in a state of invading into the outer side (outer peripheral surface) of the first resin layer 50. It is noted that the coil body 20 is not arranged in the second reduced diameter portion 14 and the second increased diameter portion 15 of the core wire 10.

The coil body 20 of the present embodiment is a loosely wound single-thread coil formed by winding one wire 21 into a single thread with a gap formed between adjacent strands of the wire 21. It is noted that the coil body 20 may be a multi-thread coil formed by winding a plurality of wires 21 into a multi-thread, or may be a single-thread twisted-wire coil formed by winding a twisted wire obtained by twisting a plurality of wires 21 into a single thread, or may be a multi-thread twisted-wire coil formed by winding each twisted wire into multiple threads using a plurality of twisted wires obtained by twisting a plurality of wires 21. It is possible to freely determine an outer diameter and an inner diameter of the coil body 20.

The coil body 20 is fixed to the core wire 10 by the distal end joint portion 40 and the intermediate fixing portion 60. The distal end joint portion 40 is a member that joins the distal end portion of the coil body 20 and the distal end portion of the small diameter portion 11 of the core wire 10. The intermediate fixing portion 60 is a member that fixes a part of the coil body 20 and a part of the first increased diameter portion 13 of the core wire 10, in the vicinity of an intermediate portion in the direction of the axial line O of the coil body 20. It is noted that the guide wire 1 may be formed with a plurality of intermediate fixing portions used for fixing the coil body 20 and the core wire 10.

The first resin layer 50 is a layer of resin that coats a part of an outer side (outer peripheral surface) of the core wire 10. Specifically, the first resin layer 50 coats a part of the proximal end side of the first increased diameter portion 13 of the core wire 10. The first resin layer 50 may be formed of a resin material having a hardness higher than that of the second resin layer 30, for example, hard urethane. Here, "hardness" refers to the micro Vickers hardness measured by a micro Vickers hardness tester.

The first resin layer 50 includes an invading portion 51 and a reduced thickness portion 52. The invading portion 51 is a portion embedded with the wire 21 of the coil body 20 on the outside (outer peripheral surface), and has a substantially constant outer diameter except for a recess portion embedded with the wire 21. In a cross section illustrated in the drawings, about half of the wire 21 is embedded in the invading portion 51. However, the ratio of the wire 21 to be embedded in the invading portion 51 may be freely changed. The reduced thickness portion 52 is an end portion at the proximal end side of the first resin layer 50, in other words, a portion provided at the proximal end side of the invading portion 51, and has a thickness of the resin layer that gradually decreases from the distal end side toward the proximal end. In the present embodiment, a length L1 of the invading portion 51 in the direction of the axial line O is longer than a length L2 of the reduced thickness portion 52 in the direction of the axial line O. The lengths L1 and L2 may be freely determined.

The second resin layer 30 is a layer of resin that coats an outer side (outer peripheral surface) of the coil body 20. Specifically, the second resin layer 30 coats outer sides of each of a whole of the coil body 20 from the distal end to the proximal end, the distal end joint portion 40, and the invading portion 51 of the first resin layer 50, and does not coat an outer side of the reduced thickness portion 52 of the first resin layer 50. The second resin layer 30 may be formed of a resin material having a hardness lower than that of the first resin layer 50, for example, soft urethane.

A depressed portion 31 is formed in a part of the second resin layer 30 that coats the coil body 20 and does not coat the first resin layer 50. The depressed portion 31 is a portion in which the resin layer enters into the gap between adjacent strands of the wire 21. When the depressed portion 31 is provided, it is possible to increase a contact surface between the second resin layer 30 and the coil body 20 (specifically, the wire 21), and it is possible to improve the joint strength between the second resin layer 30 and the coil body 20. It is not required that the second resin layer 30 includes the depressed portion 31. In the second resin layer 30, in a portion coating both the coil body 20 and the first resin layer 50, the second resin layer 30 and the first resin layer 50 contact each other in a gap between adjacent strands of the wire 21.

When such a configuration is provided, in the guide wire 1 according to the present embodiment, a part of the coil body 20 (specifically, a portion at the proximal end side of the coil body 20) is configured to be embedded into the second resin layer 30 and the first resin layer 50. In the guide wire 1, an end portion 50p at the proximal end side of the first resin layer 50 is located closer to the proximal end side relative to an end portion 30p at the proximal end side of the second resin layer 30.

The core wire 10 preferably provides an antithrombotic property, flexibility, and biocompatibility, and may be formed of a material such as a stainless alloy (SUS304, SUS316, and the like), a superelastic alloy such as a nickel-titanium alloy, a piano wire, a nickel-chromium based alloy, a cobalt alloy, and tungsten. The wire 21 of the coil body 20 may be formed of, for example, a stainless alloy such as SUS304 and SUS316, a superelastic alloy such as a nickel-titanium alloy, a piano wire, a radiolucent alloy such as a nickel-chromium based alloy and a cobalt alloy, gold, platinum, tungsten, and a radiopaque alloy such as an alloy containing these elements (for example, a platinum-nickel alloy). The distal end joint portion 40 is preferably flexible and may be formed of, for example, a resin material such as polyurethane and polyurethane elastomer. The intermediate fixing portion 60 may be formed of any joining agent, for example, a metal solder such as silver solder, gold solder, zinc, Sn-Ag alloy, and Au-Sn alloy, or an adhesive such as an epoxy adhesive. It is noted that the materials of each of the above-mentioned members are merely examples, and each member may be formed of a well-known material other than the materials mentioned above.

The guide wire 1 may be produced, for example, as follows. Firstly, the first resin layer 50 is formed in a part of the first increased diameter portion 13 of the core wire 10. Next, a freely chosen joining agent such as an epoxy adhesive is applied to an outer side (outer peripheral surface) of the invading portion 51 of the first resin layer 50. Subsequently, the wire 21 is wound around the outer sides of the small diameter portion 11 to the first increased diameter portion 13 of the core wire 10 and the invading portion 51 of the first resin layer 50 to form the coil body 20. Next, the distal end joint portion 40 and the intermediate fixing portion 60 are formed. Subsequently, a freely chosen joining agent such as an epoxy adhesive is applied to outer sides (outer peripheral surfaces) of the coil body 20, the distal end joint portion 40, and the invading portion 51 of the first resin layer 50. Next, the second resin layer 30 is formed on outer sides (outer peripheral surfaces) of the coil body 20, the distal end joint portion 40, and the invading portion 51 of the first resin layer 50. Finally, caulking is applied to the second resin layer 30 from an outer side to bring the second resin layer 30 into close contact with the coil body 20, the distal end joint portion 40, and the first resin layer 50. It is noted that the caulking may be omitted.

FIG. 2 is an enlarged view of a part pa (FIG. 1) of the guide wire 1. As described above, the guide wire 1 has a configuration in which a part of the coil body 20 is embedded in the first resin layer 50 and the second resin layer 30 (FIG. 1: a portion of the length L1). That is, as illustrated in FIG. 2, the guide wire 1 includes, on a contact surface in which each of the first resin layer 50 and the second resin layer 30 contacts the coil body 20, a portion P1 connected via the coil body 20 (hereinafter, also referred to as "indirectly connected portion") and a portion P2 in which the first resin layer 50 and the second resin layer 30 are directly connected on the contact surfaces of the first and second resin layers 50 and 30 (hereinafter, also referred to as "directly connected portion"). In the above-described manufacturing method of the guide wire 1, the caulking is applied to the second resin layer 30 from the outer side. Therefore, in the guide wire 1, the first resin layer 50 and the second resin layer 30 are connected by a chemical connecting force (connecting force by a joining agent) at the indirectly connected portion P1, a chemical connecting force (connecting force by a joining agent) at the directly connected portion P2, and a mechanical connecting force at portions P3 facing each other via the coil body 20.

As described above, the guide wire 1 of the first embodiment includes the indirectly connected portion P1 and the directly connected portion P2, and thus, as compared to a configuration including only the directly connected portion, it is possible to increase an area of the connected portion (in other words, a contact surface between members contributing to the connection of the first resin layer 50 and the second resin layer 30) and it is possible to improve a connection strength between the first resin layer 50 and the second resin layer 30. When the indirectly connected portion P1 in which the first resin layer 50 and the second resin layer 30 are connected via the coil body 20 is provided, even if shear stress SS is applied along a longitudinal direction (FIG. 2: X-axial direction) of the guide wire 1, the first resin layer 50 and the second resin layer 30 are not easily separated. Only a part of the coil body 20 is embedded into the first resin layer 50 and the second resin layer 30 (FIG. 1: a portion of the length L1), and thus, as compared to a configuration in which the entire coil body 20 from the distal end to the proximal end is embedded in the resin layer, it is possible to provide flexibility to bending. As a result, according to the guide wire 1 of the present embodiment, it is possible to provide a guide wire 1 having flexibility, being less likely to break, and providing improved safety. In the present embodiment, "breakage" is used as a general term when a part of a constituent member of the guide wire peels off, members are separated from each other, or a certain member is broken.

In the guide wire 1 according to the first embodiment, the second resin layer 30 has a lower hardness than the first resin layer 50, and thus, it is possible to flexibly configure the second resin layer 30 and the coil body 20 coated with the second resin layer 30, and it is possible to further improve the flexibility of the guide wire 1. The hardness of the first resin layer 50 is equal to or higher than the hardness of the second resin layer 30, and thus, it is possible to cause the first resin layer 50 to function as a protective member that protects a part of the coil body 20 and a part of the core wire 10.

In the guide wire 1 of the first embodiment, the end portion 50p at the proximal end side of the first resin layer 50 is located closer to the proximal end side of the core wire 10 relative to the end portion 30p at the proximal end side of the second resin layer 30 (FIG. 1). Therefore, it is possible to cause the first resin layer 50 to function as a protective member that protects the end portion at the proximal end side of the coil body 20 and a part of the core wire 10 corresponding to the proximal end portion of the coil body 20. When the first resin layer 50 includes the reduced thickness portion 52, the end portion at the proximal end side of the first resin layer 50 decreases in thickness toward the proximal end side of the core wire 10. Thus, it is possible to suppress catching on an additional device such a catheter and an endoscope and improve the slidability with respect to the additional device.

### <Second Embodiment>

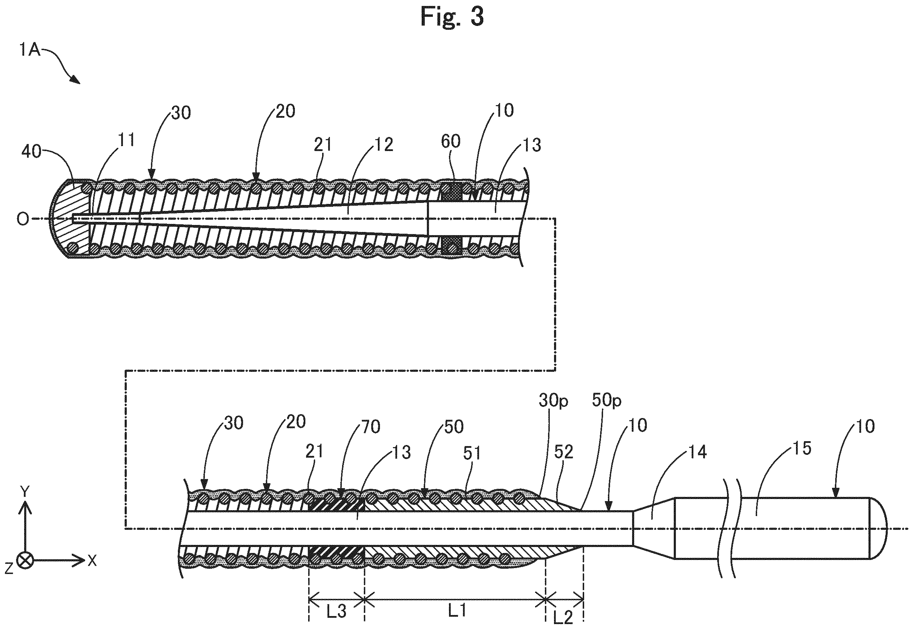

FIG. 3 is an explanatory diagram illustrating an example of a configuration of a guide wire 1A according to a second embodiment. The guide wire 1A of the second embodiment further includes a regulation portion 70 in addition to the configuration described in the first embodiment. The regulation portion 70 is provided between the coil body 20 and the core wire 10 (the first increased diameter portion 13) at the distal end side from the first resin layer 50. The wire 21 of the coil body 20 is embedded in an outer side (outer peripheral surface) of the regulation portion 70. The regulation portion 70 has a substantially constant outer diameter except for a recess portion in which the wire 21 is embedded. In the present embodiment, a length L3 of the regulation portion 70 in the direction of the axial line O is shorter than the length L1 of the invading portion 51 of the first resin layer 50, and longer than the length L2 of the reduced thickness portion 52. The regulation portion 70 may be formed by any joining agent, for example, a metal solder such as silver solder, gold solder, zinc, Sn-Ag alloy, and Au-Sn alloy, or an adhesive such as an epoxy adhesive.

As described above, the configuration of the guide wire 1A can be changed in various ways, and the regulation portion 70 may be provided. In the illustrated example, a surface at the proximal end side of the regulation portion 70 contacts a surface at the distal end side of the first resin layer 50, but the regulation portion 70 may be provided with a distance from the first resin layer 50. It is possible to freely determine the length L3 in which the regulation portion 70 is provided, and it is also possible to freely change a magnitude relationship between the lengths L1 and L2. Such a guide wire 1A according to the second embodiment exhibits an effect similar to that of the first embodiment.

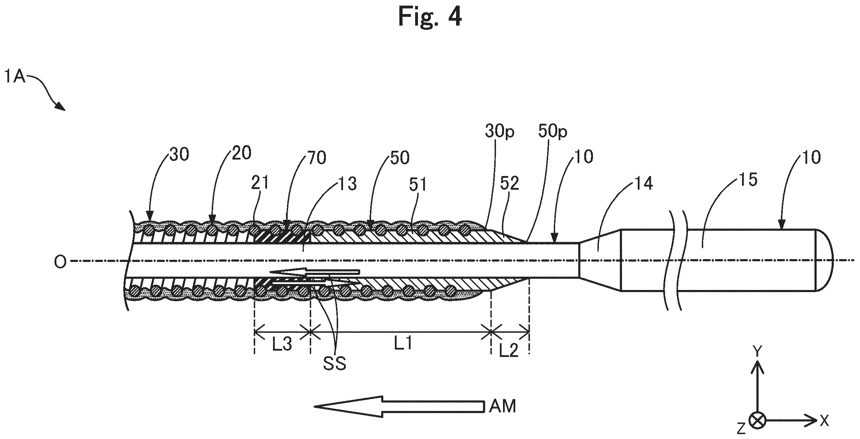

FIG. 4 is an explanatory diagram illustrating a part of the guide wire 1A at the proximal end side according to the second embodiment. The guide wire 1A according to the second embodiment includes the regulation portion 70 arranged between the coil body 20 and the core wire 10 at the distal end side from the first resin layer 50, the regulation portion 70 having another part (FIG. 3: a portion of the length L3) of the coil body 20 being embedded therein. Thus, when the shear stress SS along the longitudinal direction (X-axial direction) is applied to the guide wire 1A, if a movement of the wire 21 (the wire 21 configuring the coil body 20) is regulated by the regulation portion 70, it is possible to retain a range where the wire 21 moves in a range AM at the proximal end side from the regulation portion 70. As a result, it is possible to provide the guide wire 1A having enhanced resistance to breakage and improved safety.

### <Third Embodiment>

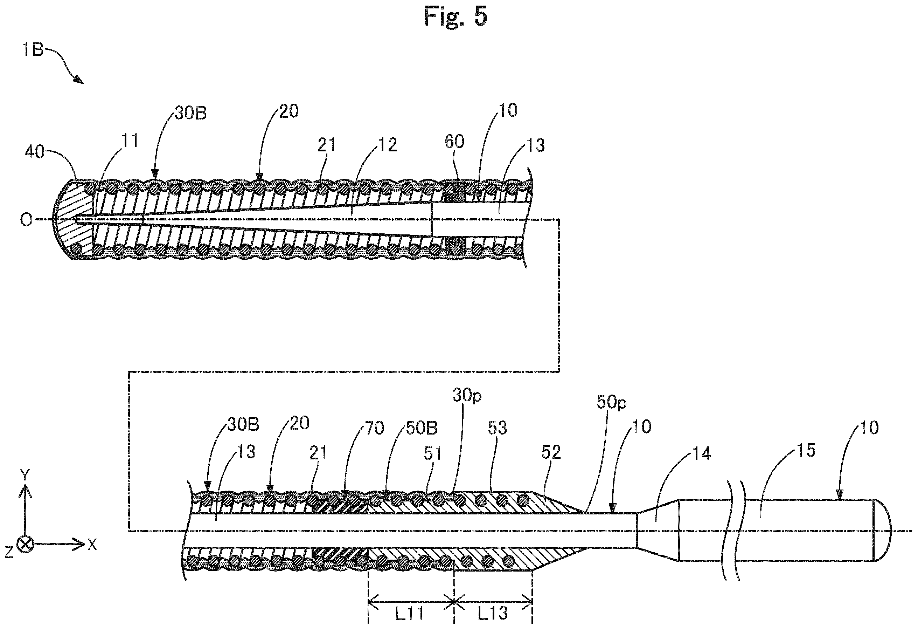

FIG. 5 is an explanatory diagram illustrating an example of a configuration of a guide wire 1B according to a third embodiment. The guide wire 1B according to the third embodiment includes a first resin layer 50B instead of the first resin layer 50, and a second resin layer 30B instead of the second resin layer 30, in the configuration described in the second embodiment.

The first resin layer 50B includes an inclusion portion 53 at the proximal end side from the invading portion 51 and at the distal end side from the reduced thickness portion 52 (in other words, between the invading portion 51 and the reduced thickness portion 52). In the inclusion portion 53, all of the proximal end portion of the coil body 20, in other words, all of the wire 21 forming the coil body 20 at the proximal end portion of the coil body 20, are embedded. The inclusion portion 53 has a substantially constant outer diameter, and the outer diameter of the inclusion portion 53 is substantially the same as a portion having the largest outer diameter of the second resin layer 30B. In the illustrated example, a length L13 of the inclusion portion 53 in the direction of the axial line O is substantially the same as a length L11 of the invading portion 51. The second resin layer 30B is not provided in an outer side (outer peripheral surface) of the inclusion portion 53, and the outer area of the inclusion portion 53 is exposed to the outside. That is, the second resin layer 30B is arranged only at the distal end side from the distal end of the inclusion portion 53.

As described above, the configuration of the guide wire 1B can be changed in various ways, and the first resin layer 50B may include the inclusion portion 53 in which all of the proximal end portion of the coil body 20 is embedded. In the illustrated example, the reduced thickness portion 52 in which the resin layer gradually decreases in thickness, is provided at the proximal end side of the inclusion portion 53 having a substantially constant outer diameter, but the inclusion portion 53 and the reduced thickness portion 52 may be integrally configured. That is, a configuration may be such that the thickness of the resin layer gradually decreases in the inclusion portion 53. An outer side (outer peripheral surface) of the inclusion portion 53 may be covered with the second resin layer 30B. Such a guide wire 1B according to the third embodiment exhibits an effect similar to those of the above-described first and second embodiments.

According to the guide wire 1B of the third embodiment, the first resin layer 50B includes, at the proximal end side, the inclusion portion 53 having all of the proximal end portion of the coil body 20 being embedded into the first resin layer 50B. Therefore, when the hardness of the first resin layer 50B is configured to be equal to or higher than the hardness of the second resin layer 30B, it is possible to improve a protection capability of protecting the end portion at the proximal end side of the coil body 20 and a part of the core wire 10 corresponding to the end portion at the proximal end side of the coil body 20. Processing for gradually decreasing the thickness of the resin layer of the end portion at the proximal end side of the first resin layer 50B toward the proximal end side, is easier.

### <Fourth Embodiment>

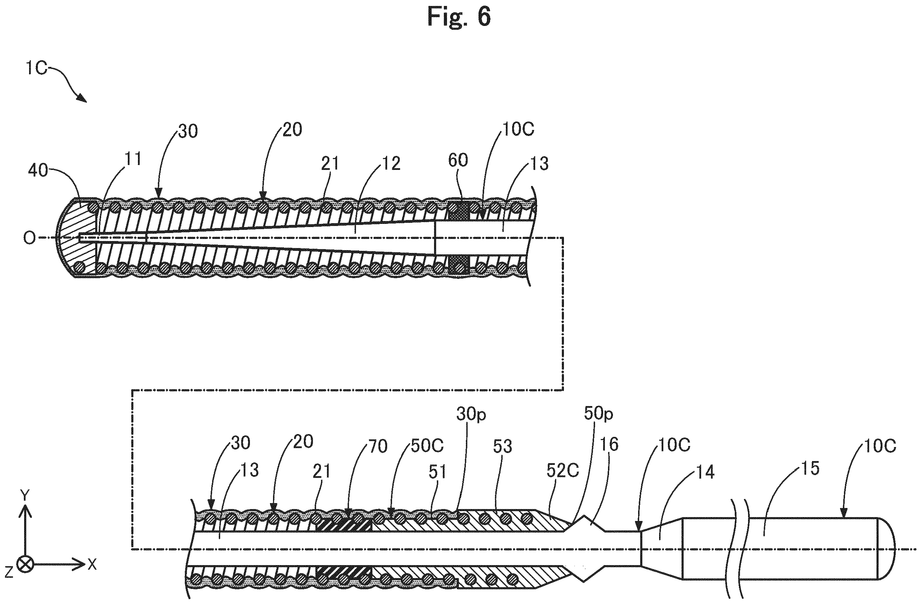

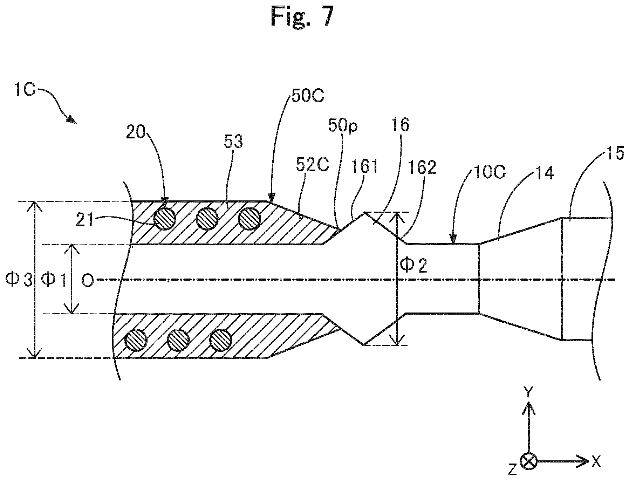

FIG. 6 is an explanatory diagram illustrating an example of a configuration of a guide wire 1C according to a fourth embodiment. FIG. 7 is an enlarged view illustrating an area in the vicinity of a protrusion portion 16 of the guide wire 1C according to the fourth embodiment. The guide wire 1C of the fourth embodiment includes a core wire 10C instead of the core wire 10 and a first resin layer 50C instead of the first resin layer 50B, in the configuration described in the third embodiment.

The core wire 10C further includes the protrusion portion 16 in addition to the small diameter portion 11 to the second increased diameter portion 15 described in the first embodiment. The protrusion portion 16 is provided in the vicinity of the end portion at the proximal end side of the first increased diameter portion 13 and at a position contacting the end portion 50p at the proximal end side of the first resin layer 50C. As illustrated in FIG. 7, an outer diameter phi 2 of the protrusion portion 16 in a portion where the outer diameter of the protrusion portion 16 is largest is larger than an outer diameter phi 1 of the first increased diameter portion 13 and smaller than an outer diameter phi 3 of the inclusion portion 53 of the first resin layer 50C. As illustrated in FIG. 7, the protrusion portion 16 includes a reduced diameter portion 161 having an outer diameter that decreases from phi 2 to phi 1 from the proximal end side to the distal end side, and an increased diameter portion 162 having an outer diameter that increases from phi 1 to phi 2 from the proximal end side to the distal end side. The first resin layer 50C includes a reduced thickness portion 52C instead of the reduced thickness portion 52, in the configuration described in the third embodiment. In the reduced thickness portion 52C, the end portion 50p at the proximal end side is located on the reduced diameter portion 161 of the protrusion portion 16. That is, the end portion 50p at the proximal end side of the first resin layer 50C is provided in contact with the reduced diameter portion 161 of the protrusion portion 16.

As described above, the configuration of the guide wire 1C can be changed in various ways, and it is possible to adopt a configuration in which the core wire 10C including the protrusion portion 16 is provided and the end portion 50p at the proximal end side of the first resin layer 50C is protected by the protrusion portion 16. The outer diameter phi 2 of the protrusion portion 16 can be freely changed as long as such a diameter phi 2 is larger than the outer diameter phi 1 of the first increased diameter portion 13 and smaller than the outer diameter phi 3 of the inclusion portion 53. Such a guide wire 1C according to the fourth embodiment exhibits an effect similar to those of the above-described first to third embodiments.

According to the guide wire 1C of the fourth embodiment, the core wire 10C includes the protrusion portion 16 having an enlarged diameter at a position contacting the end portion 50p at the proximal end side of the first resin layer 50C. Therefore, it is possible to protect the end portion 50p at the proximal end side of the first resin layer 50C with the protrusion portion 16 of the core wire 10C, and it is possible to suppress peeling of the proximal end portion 50p of the first resin layer 50C. It is further possible to regulate a movement of the wire 21 (the wire 21 configuring the coil body 20) when the shear stress along the longitudinal direction (X-axial direction) is applied to the guide wire 1C, by the protrusion portion 16 of the core wire 10C. As a result, it is possible to provide a guide wire 1C having enhanced resistance to breakage and improved safety.

According to the guide wire 1C of the fourth embodiment, the end portion 50p at the proximal end side of the first resin layer 50C is provided in contact with the reduced diameter portion 161 of the protrusion portion 16. Therefore, it is possible to surely protect the end portion 50p at the proximal end side of the first resin layer 50C, and it is possible to reduce a possibility that kinking occurs in the core wire 10C, by suppressing concentration of stress applied to the protrusion portion 16 when a shear stress along the longitudinal direction (X-axial direction) is applied to the guide wire 1C. It is possible to suppress generation of a step as a result of exposure of the core wire 10C at the distal end side from the protrusion portion 16, and thus, it is possible to suppress catching on the additional device and improve the slidability with respect to the additional device.

### <Comparative Examples>

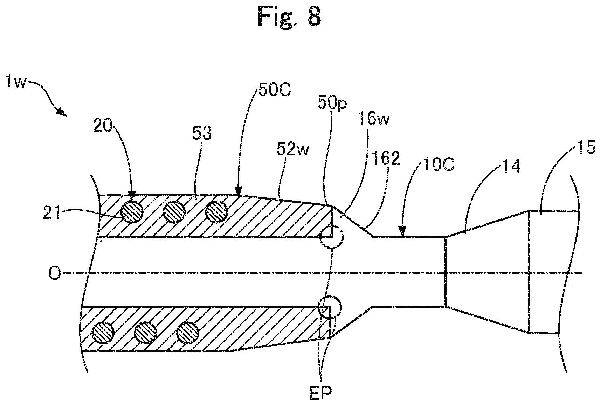

FIG. 8 is an enlarged view illustrating an area in the vicinity of a protrusion portion 16w of a guide wire 1w according to a comparative example. The guide wire 1w of the comparative example includes a protrusion portion 16w instead of the protrusion portion 16 and a reduced thickness portion 52w instead of the reduced thickness portion 52C, in the configuration of the fourth embodiment. The protrusion portion 16w does not include the reduced diameter portion 161 described in the fourth embodiment, and the proximal end portion 50p of the reduced thickness portion 52w of the first resin layer 50C is arranged in a portion where an outer diameter of the protrusion portion 16w is largest. In such a guide wire 1w of the comparative example, kinking may possibly occur in the core wire 10C as a result of the stress being concentrated at a corner portion EP of the protrusion portion 16w when the shear stress along the longitudinal direction (X-axial direction) is applied to the guide wire 1w. Therefore, as described in the fourth embodiment, the end portion 50p at the proximal end side of the first resin layer 50C is preferably provided in contact with the reduced diameter portion 161 of the protrusion portion 16.

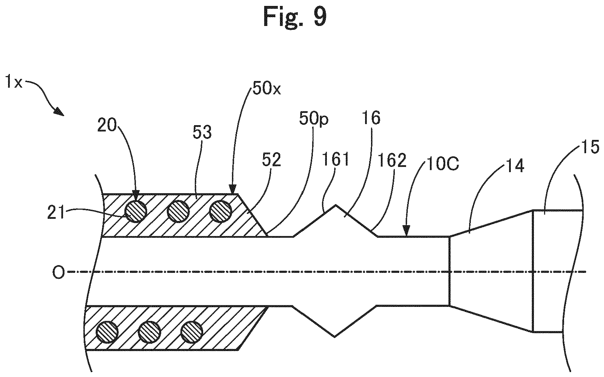

FIG. 9 is an enlarged view illustrating an area in the vicinity of the protrusion portion 16 of a guide wire 1x according to a comparative example. The guide wire 1x of the comparative example includes a first resin layer 50x instead of the first resin layer 50, in the configuration of the fourth embodiment. The end portion 50p at the proximal end side of the first resin layer 50x (in other words, the end portion 50p at the proximal end side of the reduced thickness portion 52 of the first resin layer 50x) is arranged in the first increased diameter portion 13, and is not arranged in contact with the reduced diameter portion 161 of the protrusion portion 16. In such a guide wire 1x of the comparative example, the first increased diameter portion 13 is exposed at the distal end side from the protrusion portion 16, and thus, there is a possibility that the protrusion portion 16 is caught on an additional device. In other words, in the guide wire 1x of the comparative example, there is room for improvement in terms of slidability with respect to the additional device. Therefore, as described in the fourth embodiment, the end portion 50p at the proximal end side of the first resin layer 50C is preferably provided in contact with the reduced diameter portion 161 of the protrusion portion 16.

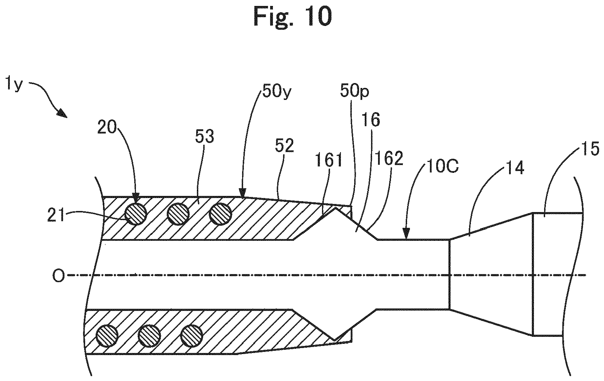

FIG. 10 is an enlarged view illustrating an area in the vicinity of the protrusion portion 16 of a guide wire 1y according to a comparative example. The guide wire 1y of the comparative example includes a first resin layer 50y instead of the first resin layer 50, in the configuration of the fourth embodiment. The end portion 50p at the proximal end side of the first resin layer 50y (in other words, the end portion 50p at the proximal end side of the reduced thickness portion 52 of the first resin layer 50x) is arranged in contact with the increased diameter portion 162 of the protrusion portion 16. In such a guide wire 1y of the comparative example, it is not possible to protect the end portion 50p at the proximal end side of the first resin layer 50y by the protrusion portion 16, and thus, there is a possibility that peeling may occur at the end portion 50p at the proximal end side of the first resin layer 50y. Therefore, as described in the fourth embodiment, the end portion 50p at the proximal end side of the first resin layer 50C is preferably provided in contact with the reduced diameter portion 161 of the protrusion portion 16.

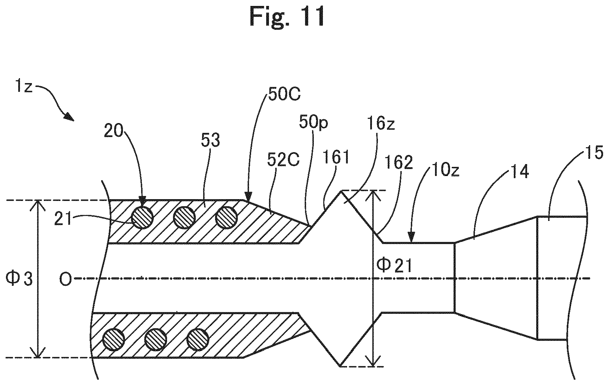

FIG. 11 is an enlarged view illustrating an area in the vicinity of a protrusion portion 16z of a guide wire 1z according to a comparative example. The guide wire 1z of the comparative example includes the protrusion portion 16z instead of the protrusion portion 16, in the configuration of the fourth embodiment. An outer diameter phi 21 of the protrusion portion 16z in a portion where an outer diameter of the protrusion portion 16z is largest is larger than the outer diameter phi 3 of the inclusion portion 53 of the first resin layer 50C. In such a guide wire 1z of the comparative example, the protrusion portion 16z may possibly be caught by an additional device. In other words, in the guide wire 1z of the comparative example, there is room for improvement in terms of slidability with respect to the additional device. Therefore, as described in the fourth embodiment, the outer diameter phi 2 of the protrusion portion 16 is preferably smaller than the outer diameter phi 3 of the inclusion portion 53 of the first resin layer 50C.

### <Fifth Embodiment>

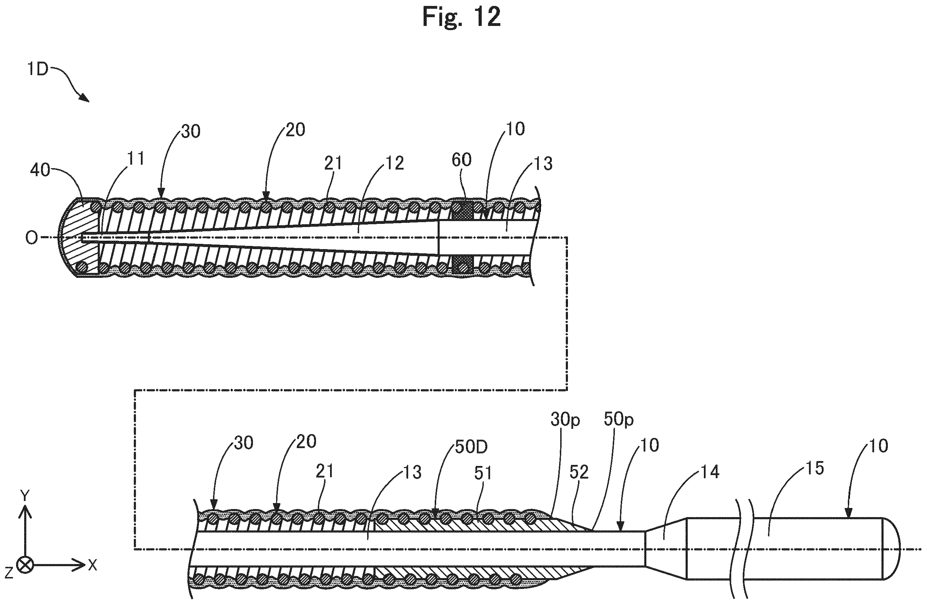

FIG. 12 is an explanatory diagram illustrating a configuration of a guide wire 1D according to a fifth embodiment. The guide wire 1D of the fifth embodiment includes a first resin layer 50D instead of the first resin layer 50, in the configuration described in the first embodiment. The first resin layer 50D may be formed of a resin material having a hardness equal to that of the second resin layer 30 or lower than that of the second resin layer 30, for example, soft urethane. As described above, the configuration of the guide wire 1D can be changed in various ways, and the first resin layer 50D and the second resin layer 30 may be formed of the same material. Such a guide wire 1D according to the fifth embodiment exhibits an effect similar to that of the first embodiment.

### <Sixth Embodiment>

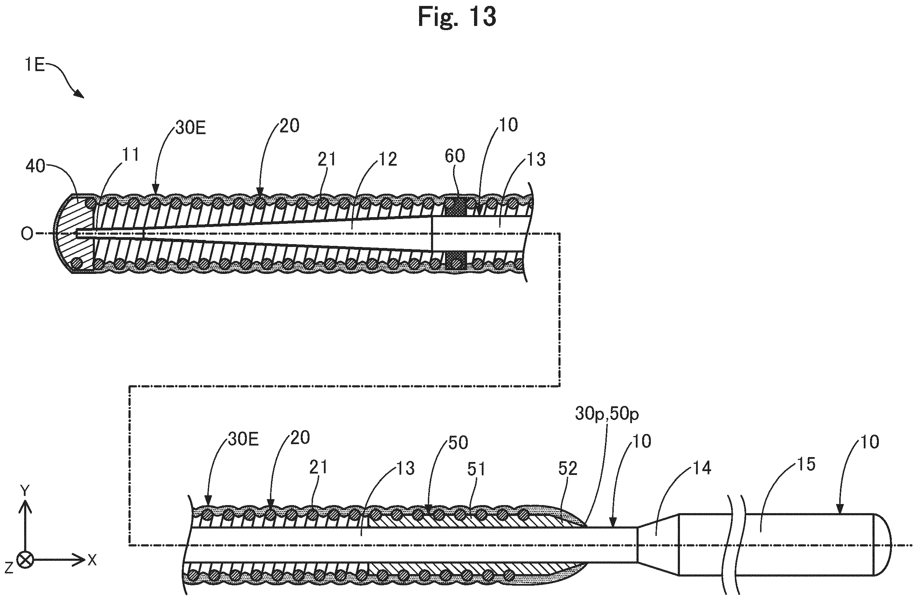

FIG. 13 is an explanatory diagram illustrating an example of a configuration of a guide wire 1E according to a sixth embodiment. The guide wire 1E of the sixth embodiment includes a second resin layer 30E instead of the second resin layer 30, in the configuration described in the first embodiment. In the second resin layer 30E, the end portion 30p at the proximal end side is arranged at substantially the same position as the end portion 50p at the proximal end side of the first resin layer 50. That is, the second resin layer 30E covers the entire outer side (outer peripheral surface) of the first resin layer 50 (the invading portion 51 and the reduced thickness portion 52). As described above, the configuration of the guide wire 1E can be changed in various ways, and the end portion 30p at the proximal end side of the second resin layer 30E may be arranged at substantially the same place as the end portion 50p at the proximal end side of the first resin layer 50, or at the proximal end side from the end portion 50p at the proximal end side of the first resin layer 50. Such a guide wire 1E according to the sixth embodiment exhibits an effect similar to that of the first embodiment.

### <Seventh Embodiment>

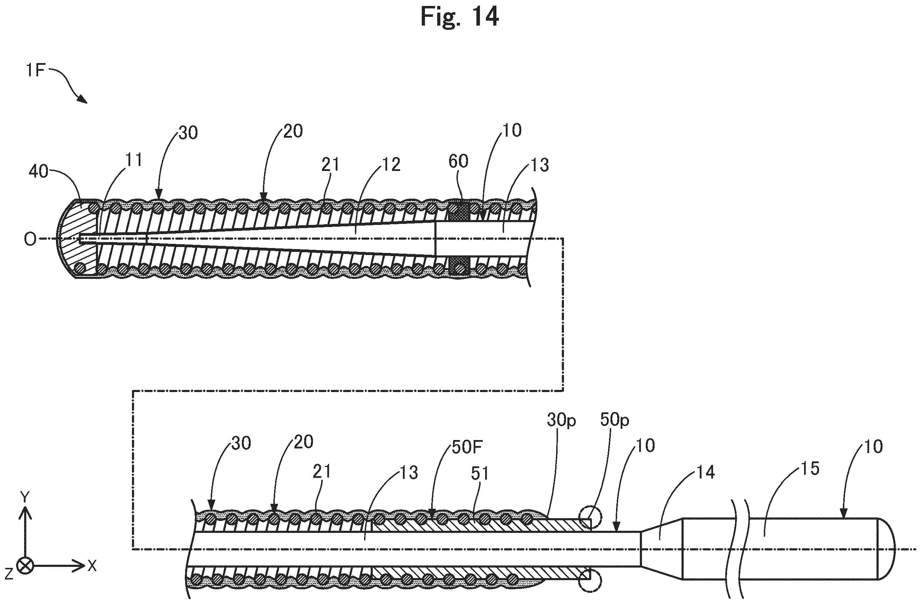

FIG. 14 is an explanatory diagram illustrating an example of a configuration of a guide wire 1F according to a seventh embodiment. The guide wire 1F of the seventh embodiment includes a first resin layer 50F instead of the first resin layer 50, in the configuration described in the first embodiment. The first resin layer 50F does not include the reduced thickness portion 52 described in the first embodiment. As described above, the configuration of the guide wire 1F can be changed in various ways, and the first resin layer 50F not including the reduced thickness portion 52 may be used. A corner portion at the proximal end side of the first resin layer 50F (FIG. 14: dotted line circle frame) may be attached at a freely chosen angle R. Such a guide wire 1F according to the seventh embodiment exhibits an effect similar to that of the first embodiment.

### <Eighth Embodiment>

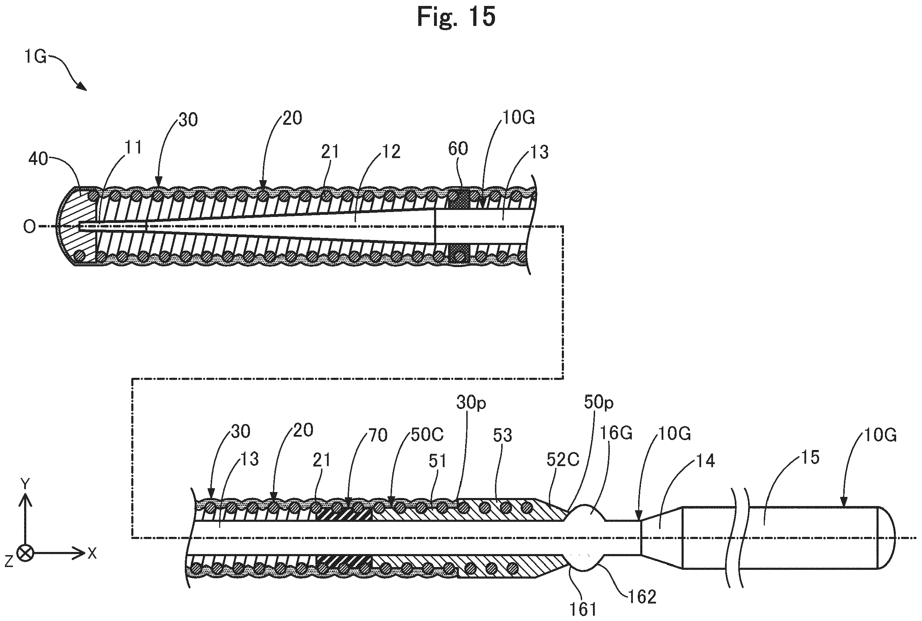

FIG. 15 is an explanatory diagram illustrating an example of a configuration of a guide wire 1G according to an eighth embodiment. The guide wire 1G of the eighth embodiment includes a core wire 10G instead of the core wire 10C in the configuration described in the fourth embodiment. The core wire 10G includes a protrusion portion 16G instead of the protrusion portion 16. In the protrusion portion 16G, the reduced diameter portion 161 and the increased diameter portion 162 are gradually connected to form a rounded shape. In other words, the protrusion portion 16G has a substantially semi-circular shape in the cross section illustrated in FIG. 15. Such a guide wire 1G according to the eighth embodiment exhibits an effect similar to those of the above-described first to fourth embodiments.

### <Modifications of Present Embodiment>

The disclosed embodiments are not limited to the above-described embodiments, and may be implemented in various modes without departing from the spirit of the disclosed embodiments. The following modifications can be applied, for example.

### [First Modification]

In the above-described first to eighth embodiments, examples of the configurations of the guide wires 1 and 1A to 1G are described. However, the configuration of the guide wire 1 can be variously modified. For example, it is not required that the guide wire 1 includes the small diameter portion 11 and the first reduced diameter portion 12. For example, the core wire 10 of the guide wire 1 may be formed by a plurality of core wires formed by using different materials. For example, it is not required that the second resin layer 30 covers the distal end joint portion 40, or covers the entire coil body 20. For example, the first resin layer 50 may be integrally configured with the second resin layer 30. For example, the end portion 50p at the proximal end side of the first resin layer 50 may be provided in contact with the second reduced diameter portion 14 of the core wire 10.

### [Second Modification]

The configurations of the guide wires 1 and 1A to 1G of the first to eighth embodiments described above, and the configurations of the guide wires 1 and 1A to 1G of the modification described above may be appropriately combined. For example, in the configuration of the first embodiment, the inclusion portion 53 (third embodiment) may be provided, or the protrusion portion 16 (fourth and eighth embodiments) may be provided. For example, in the configurations described in the second, third, and fourth embodiments, the first resin layer 50D of the fifth embodiment may be used, the second resin layer 30E of the sixth embodiment may be used, and the first resin layer 50F of the seventh embodiment may be used.

Although the aspects have been described based on the embodiments and the modifications, the embodiments of the above-described aspects are for facilitating understanding of the aspects, and do not limit the aspects. The aspects can be modified and improved without departing from the spirit of the aspects and the scope of the claims, and equivalent aspects are included in the aspects. Further, unless a technical feature is described as essential in the present specification, the technical feature may be omitted as appropriate.

### DESCRIPTION OF REFERENCE NUMERALS

1, 1A to 1G, 1w to 1z Guide wire
10, 10C, 10G Core wire
11 Small diameter portion
12 First reduced diameter portion
13 First increased diameter portion
14 Second reduced diameter portion
15 Second increased diameter portion
16, 16G, 16w, 16z Protrusion portion
20 Coil body
21 Wire
30, 30B, 30E Second resin layer
31 Depressed portion
40 Distal end joint portion
50, 50B to 50D, 50F, 50x, 50y First resin layer
51 Invading portion
52, 52C, 52w Reduced thickness portion
53 Inclusion portion
60 Intermediate fixing portion
70 Regulation portion
161 Reduced diameter portion
162 Increased diameter portion

## Claims

1. A guide wire comprising:
a core wire;
a first resin layer that coats a part of the core wire;
a coil body wound around another part of the core wire and an outer side of the first resin layer; and
a second resin layer that coats the coil body, wherein
the part of the coil body is embedded in the first resin layer and the second resin layer.

2. The guide wire according to claim 1, wherein
the second resin layer is lower in hardness than the first resin layer.

3. The guide wire according to claim 1 or 2, wherein
an end portion at a proximal end side of the first resin layer is located close to a proximal end side of the core wire relative to an end portion at a proximal end side of the second resin layer.

4. The guide wire according to any one of claims 1 to 3, wherein
the end portion at the proximal end side of the first resin layer decreases in thickness toward the proximal end side of the core wire.

5. The guide wire according to any one of claims 1 to 4, wherein
the first resin layer includes, at the proximal end side, an inclusion portion having all of the proximal end portion of the coil body being embedded into the first resin layer, and
the second resin layer is arranged only at a distal end side from a distal end of the inclusion portion.

6. The guide wire according to any one of claims 1 to 5, wherein
the core wire includes a protrusion portion having an enlarged diameter at a position contacting the end portion at the proximal end side of the first resin layer.

7. The guide wire according to claim 6, wherein
the protrusion portion includes a reduced diameter portion having an outer diameter decreasing from a proximal end side toward a distal end side, and
the end portion at the proximal end side of the first resin layer is provided in contact with the reduced diameter portion.

8. The guide wire according to any one of claims 1 to 7, further comprising:
a regulation portion arranged between the coil body and the core wire at a distal end side from the first resin layer, the regulation portion having another part of the coil body being embedded therein.
